(19)

**Europäisches Patentamt**
**European Patent Office**
**Office européen des brevets**

(11) **EP 1 970 351 A1**

(12) **EUROPEAN PATENT APPLICATION**

(43) Date of publication:
**17.09.2008 Bulletin 2008/38**

(51) Int Cl.:
*C01B 37/08* [(2006.01)]     *B01J 29/85* [(2006.01)]
*C07C 1/20* [(2006.01)]     *C07C 1/26* [(2006.01)]
*C07C 1/32* [(2006.01)]

(21) Application number: **07300861.7**

(22) Date of filing: **13.03.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicants:
• **TOTAL PETROCHEMICALS RESEARCH FELUY**
  **7181 Seneffe (Feluy) (BE)**
• **CENTRE NATIONAL DE**
  **LA RECHERCHE SCIENTIFIQUE (CNRS)**
  **Etablissement Public**
  **75016 Paris (FR)**

(72) Inventors:
• **Vermeiren, Walter**
  **3530 Houthalen (BE)**
• **Nesterenko, Nikolai**
  **1400 Nivelles (BE)**
• **Petitto, Carolina, Résidence de Mail des Abbés C1**
  **34000 Montpellier (FR)**
• **Di Renzo, Francesco, Les Cèdres BC**
  **34070 Montpellier (FR)**
• **Fajula, Francois**
  **34820 Teyran (FR)**

(74) Representative: **Leyder, Francis**
  **Total Petrochemicals Research Feluy**
  **Zone Industrielle C**
  **7181 Seneffe (Feluy) (BE)**

(54) **Method of preparing metalloaluminophosphate (Meapo) molecular sieve**

(57)    The present invention also relates to a method for preparing metalloaluminophosphate (MeAPO) molecular sieve said method comprising :

a) forming a reaction mixture containing a texture influencing agent (TIA), an organic templating agent (TEMP), at least a reactive inorganic source of $MeO_2$ insoluble in the TIA, reactive sources of $Al_2O_3$ and $P_2O_5$,
b) crystallizing the above reaction mixture thus formed until crystals of the metalloaluminophosphate are formed,
c) recovering a solid reaction product,
d) washing it with water to remove the TIA and
e) calcinating it to remove the organic template.

In a usual embodiment said reaction mixture has a composition expressed in terms of molar oxide ratios of :
$TEMP/Al_2O_3$ = 0.3-5 , more desirable 0.5-2
$MeO_2/Al_2O_3$ = 0.005-2.0, more desirable 0.022-0.8
$P_2O_5/Al_2O_3$ =0.5-2, more desirable 0.8-1.2
$TIA/Al_2O_3$ = 3-30, more desirable 6-20

In a usual embodiment the metalloaluminophosphate (MeAPO) molecular sieves made with the above method have a lamellar crystal morphology having an empirical chemical composition on an anhydrous basis, after synthesis and calcination, expressed by the formula $H_xMe_yAl_zP_kO_2$ wherein, y+z+k=1, x<=y
y has a value ranging from 0.0008 to 0.4 and more desirable from 0.005 to 0.18
z has a value ranging from 0.25 to 0.67 and more desirable from 0.38 to 0.55
k has a value ranging from 0.2 to 0.67 and more desirable from 0.36 to 0.54
said molecular sieve having predominantly a plate crystal morphology.

In an advantageous embodiment the MeAPO made by the method of the invention have essentially a structure CHA or AEI or a mixture thereof. Preferably they have essentially the structure SAPO 18 or SAPO 34 or a mixture thereof.

The present invention also relates to catalysts consisting of the above MeAPO molecular sieves made by the method of the invention or comprising the above MeAPO molecular sieves made by the method of the invention.

The present invention also relates to a process for making an olefin product from an oxygen-containing, halogenide-containing or sulphur-containing organic feedstock wherein said oxygen-containing, halogenide-containing or sulphur-containing organic feedstock is contacted with the above catalyst under conditions effective to convert the oxygen-containing, halogenide-containing or sulphur-containing organic feedstock to olefin products.

EP 1 970 351 A1

**Description**

[Field of the invention]

The present invention relates to a method for preparing metalloaluminophosphate (MeAPO) molecular sieve. The metalloaluminophosphate molecular sieves of the invention are useful as catalysts in a variety of processes including cracking, hydrocracking, isomerization, reforming, dewaxing, alkylation, transalkylation, conversion of methanol to light olefins. The limited supply and increasing cost of crude oil has prompted the search for alternative processes for producing hydrocarbon products. One such process is the conversion of oxygen-containing, halogenide-containing or sulphur-containing organic compounds to hydrocarbons and especially light olefins (by light olefins is meant $C_2$ to $C_4$ olefins) or gasoline and aromatics. The interest in the methanol to olefin (MTO) process is based on the fact that oxygenates, especially methanol can be obtained from coal, biomass, organic waste or natural gas by the production of synthesis gas which is then processed to produce methanol.

[Background of the invention]

US 4,440,871 describes microporous crystalline silicoaluminophosphates (referred as SAPO) the pores of which are uniform and have nominal diameters of greater than about 3 Angstroms and whose essential empirical chemical composition in the as-synthesized and anhydrous form is $mR:(Si_x Al_y P_z)O_2$ wherein "R" represents at least one organic templating agent present in the intracrystalline pore system; "m" has a value of from 0.02 to 0.3; "m" represents the moles of "R" present per mole of $(Si_x Al_y P_z)O_2$ ; "x", "y" and "z" represent the mole fractions of silicon, aluminum and phosphorus respectively, present as tetrahedral oxides, said mole fractions being such that they are within a specific area in the ternary diagram $Si_x Al_y P_z$. Process for preparing said SAPO comprises forming a reaction mixture containing reactive sources of $SiO_2$, $Al_2 O_3$, and $P_2 O_5$ and an organic templating agent, said reaction mixture having a composition expressed in terms of molar oxide ratios of: $aR_2 O:(Si_x Al_y P_z)O_2 :bH_2O$ wherein "R" is an organic templating agent; "a" has a value large enough to constitute an effective amount of "R" and is within the range of greater than 0 to 3; "b" has a value of from zero to 500; "x", "y" and "z" represent the mole fractions, respectively, of silicon, aluminum and phosphorus in the $(Si_x Al_y P_z)O_2$ constituent and each has a value of at least 0.01 and crystallizing the reaction mixture thus formed at a temperature of at least 100° C until crystals of the silicoaluminophosphate are formed.

US 6,207,872 relates to a process for converting methanol to light olefins comprising contacting the methanol with a catalyst at conversion conditions, the catalyst comprising a crystalline metallo aluminophosphate molecular sieve having a chemical composition on an anhydrous basis expressed by an empirical formula of: $(EL_xAl_yP_z)O_2$ where EL is a metal selected from the group consisting of silicon, magnesium, zinc, iron, cobalt, nickel, manganese, chromium and mixtures thereof, "x" is the mole fraction of EL and has a value of at least 0.005, "y" is the mole fraction of Al and has a value of at least 0. 01, "z" is the mole fraction of P and has a value of at least 0.01 and x+y+z=1, the molecular sieve characterized in that it has predominantly a plate crystal morphology, wherein the average smallest crystal dimension is at least 0.1 micron and has an aspect ratio of less than or equal to 5.

US 6,334,994 relates to a microporous crystalline silico-alumino-phosphate composition, the theoretical composition of which, on a water-free basis after synthesis and calcination, is: $H_wSi_xAl_yP_zO_2$ where w and x have a value between 0.01 and 0.05 and y and z are values between 0.4 and 0.6, wherein the composition is a mixed phase product comprising silico-alumino-phosphates of AEI and CHA structure prepared in one batch crystallization, not including mere physical mixtures, the product after calcination in air at 550° C for 4 hours, produces a specific X-ray diffractogram and XRD-profiles.

EP 893159 relates to a method for preparing catalysts comprising silica-modified crystalline silicoaluminophosphate molecular sieves, which comprises adding an aluminum alkoxide to an aqueous amine or organic ammonium salt solution cooled at a temperature of not higher than 20°C, followed by hydrolysis, until a uniform aqueous aluminum hydroxide colloid or solution is formed, adding, to the colloid or solution, silica or other Si-source compounds, and phosphoric acid or other P-source compounds, if desired, along with a metal source selected from the group of Li, Ti, Zr, V, Cr, Mn, Fe, Co, Zn, Be, Mg, Ca, B, Ga and Ge, hydrothermally treating the resulting mixture to prepare a crystalline silicoaluminophosphate molecular sieve, and then modifying the crystalline silicoaluminophosphate molecular sieve with silica.

US 2005 0096214 (US 6953767) relates to a process for making an olefin product from an oxygenate feedstock comprising contacting said oxygenate feedstock with a catalyst comprising a silicoaluminophosphate molecular sieve comprising at least one intergrown phase of molecular sieves having AEI and CHA framework types, wherein said intergrown phase has an AEI/CHA ratio of from about 5/95 to 40/60 as determined by DIFFaX analysis, using the powder X-ray diffraction pattern of a calcined sample of said silicoaluminophosphate molecular sieve, under conditions effective to form an olefin product.

It also describes a method for preparing the molecular sieve of said process that comprises

(a) combining a reactive source of silicon, a reactive source of phosphorus and a hydrated aluminum oxide in the presence of an organic structure directing agent (template) to form a mixture;

(b) mixing and heating continuously the mixture prepared at step a) up to the crystallization temperature;

(c) maintaining the mixture at the crystallization temperature and under stirring for a period of time of from 2 to 150 hours;

(d) recovering crystals of the silicoaluminophosphate molecular sieve

(e) wherein the mixture prepared at step a) has a molar composition within the following ranges:

$P_2O_5$:$Al_2O_3$ from 0.6:1 to 1.2:1

$SiO_2$:$Al_2O_3$ from 0.005:1 to 0.35:1

$H_2O$:$Al_2O_3$ from 10:1 to 40:1

and the template is a tetraethylammonium compound.

In all the above prior arts only template and/or specific reaction conditions are used to influence the crystal structure of the material. It has been discovered that preparing said MeAPO in the presence of one template, one texture influencing agent (a kind of template), inorganic metal source insoluble in the texture influencing agent, Al and P source, all these ingredients being in specific proportions, MeAPO with high efficiency in MTO process are obtained. The template can be tetraethylammonium hydroxide (TEAOH) or an amine. The texture influencing agent can be an alcohol, a diol or glycerol.

US 6,540,970 relates to a method for making a metalloaluminophosphate (MeAPO) molecular sieve, said process comprising the steps of:

providing a source of alumina, a source of phosphorus, water, and a template suitable for forming a MeAPO molecular sieve;

providing a source of metal including metal particles, said metal particles measuring, in their largest dimension, equal to or less than five nanometers;

providing a water soluble organic solvent capable of solubilizing said source of metal;

forming a synthesis mixture from said source of alumina, said source of phosphorus, said water, said template, said source of metal, and said solvent;

and forming a MeAPO molecular sieve from said synthesis mixture.

Desirably, the water soluble organic solvent capable of solubilizing the source of the metal is selected from the group consisting of sulfoxides and $C_1$ to $C_5$ oxygenated hydrocarbons. Desirably, the oxygenated hydrocarbon is selected from the group consisting of alcohols (branched or normal), ketones, aldehydes, diols and acids. Useful solvents include one or more solvents selected from the group consisting of acetone, 1,2-propanediol, 1,3-propanediol, methanol, ethanol, propanol, isopropanol, butanol, and ethylene glycol. Desirably, the solvent is an alcohol. The products obtained are isocrystalline spheroidal particles comprising a SAPO molecular sieve. The particle measures from 0.5 microns to 30 microns in diameter.

This process doesn't lead to MeAPO with very thin lamellar plate crystal morphology.

[Brief summary of the invention]

The present invention relates to a method for preparing metalloaluminophosphate (MeAPO) molecular sieve said method comprising :

a) forming a reaction mixture containing a texture influencing agent (TIA), an organic templating agent (TEMP), at least a reactive inorganic source of $MeO_2$ essentially insoluble in the TIA, reactive sources of $Al_2O_3$ and $P_2O_5$,

b) crystallizing the above reaction mixture thus formed until crystals of the metalloaluminophosphate are formed,

c) recovering a solid reaction product,

d) washing it with water to remove the TIA and

e) calcinating it to remove the organic template.

In a usual embodiment said reaction mixture has a composition expressed in terms of molar oxide ratios of :

$TEMP/Al_2O_3$ = 0.3-5, more desirable 0.5-2

$MeO_2/Al_2O_3$ = 0.005-2.0, more desirable 0.022-0.8

$P_2O_5/Al_2O_3$ =0.5-2, more desirable 0.8-1.2

$TIA/Al_2O_3$ = 3-30, more desirable 6-20

In an advantageous embodiment $TEMP/Al_2O_3 = 0.5-2$ ; $MeO_2/Al_2O_3 = 0.022-0.8$; $P_2O_5/Al_2O_3 = 0.8-1.2$ and $TIA/Al_2O_3 = 6-20$.

In a first preferred embodiment $TEMP/Al_2O_3 = 0.5-2$ ; $MeO_2/Al_2O_3 = 0.022-0.7$; $P_2O_5/Al_2O_3 = 0.8-1.2$ and $TIA/Al_2O_3 = 6-20$.

In a second preferred embodiment $TEMP/Al_2O_3 = 0.7-2$ ; $MeO_2/Al_2O_3 = 0.05-0.7$; $P_2O_5/Al_2O_3 = 0.8-1.2$ and $TIA/Al_2O_3 = 6-20$.

In a third preferred embodiment $TEMP/Al_2O_3 = 0.7-2$ ; $MeO_2/Al_2O_3 = 0.05-0.6$; $P_2O_5/Al_2O_3 = 0.8-1.2$ and $TIA/Al_2O_3 = 6-20$.

The metalloaluminophosphate (MeAPO) molecular sieves made with the above method have a lamellar crystal morphology.

In a usual embodiment the metalloaluminophosphate (MeAPO) molecular sieves made with the above method have a lamellar crystal morphology having an empirical chemical composition on an anhydrous basis, after synthesis and calcination, expressed by the formula $H_xMe_yAl_zP_kO_2$ wherein,

$$y+z+k=1$$

$$x<=y$$

y has a value ranging from 0.0008 to 0.4 and more desirable from 0.005 to 0.18 z has a value ranging from 0.25 to 0.67 and more desirable from 0.38 to 0.55 k has a value ranging from 0.2 to 0.67 and more desirable from 0.36 to 0.54 said molecular sieve having predominantly a plate crystal morphology.

The values of y, z and k in the usual embodiment are obtained by the ratios of the ingredients described in the usual embodiment method above described.

In an advantageous embodiment y has a value ranging from 0.005 to 0.18, z has a value ranging from 0.38 to 0.55 and k has a value ranging from 0.36 to 0.54.

In a first preferred embodiment y has a value ranging from 0.005 to 0.16, z has a value ranging from 0.39 to 0.55 and k has a value ranging from 0.37 to 0.54.

In a second preferred embodiment y has a value ranging from 0.011 to 0.16, z has a value ranging from 0.39 to 0.55 and k has a value ranging from 0.37 to 0.54.

In a third preferred embodiment y has a value ranging from 0.011 to 0.14, z has a value ranging from 0.40 to 0.55 and k has a value ranging from 0.38 to 0.54.

The values of y, z and k in the advantageous, first, second and third embodiments described above are obtained by using the ingredients ratios described respectively in the advantageous, first, second and third embodiments of the method described above.

In an advantageous embodiment the MeAPO made by the method of the invention have essentially a structure CHA or AEI or a mixture thereof. Preferably they have essentially the structure SAPO 18 or SAPO 34 or a mixture thereof.

The present invention also relates to catalysts consisting of the above MeAPO molecular sieves made by the method of the invention or comprising the above MeAPO molecular sieves made by the method of the invention.

The present invention also relates to a process for making an olefin product from an oxygen-containing, halogenide-containing or sulphur-containing organic feedstock wherein said oxygen-containing, halogenide-containing or sulphur-containing organic feedstock is contacted with the above catalyst under conditions effective to convert the oxygen-containing, halogenide-containing or sulphur-containing organic feedstock to olefin products.

According to an advantageous embodiment of the invention said olefin products are fractionnated to form a stream comprising essentially ethylene and at least a part of said stream is recycled on the catalyst to increase the propylene production and then the flexibility of ethylene vs propylene production. Advantageously the ratio of ethylene to the oxygen-containing, halogenide-containing or sulphur-containing organic feedstock is 1.8 or less.

[Detailed description of the invention]

**With regards to the plate crystal morphology,** by predominantly is meant advantageously greater than 50% of the crystals. Preferably at least 70% of the crystals have a plate morphology and most preferably at least 90% of the crystals have a plate morphology. About "essentially" referring to the CHA or AEI structure it means that advantageously more than 80% by weight, preferably more than 90%, of the MeAPO of the invention has the structure CHA or AEI or a mixture thereof. About "essentially" referring to the SAPO 18 or SAPO 34 structure it means that advantageously more than 80% by weight, preferably more than 90%, of the MeAPO of the invention has the structure SAPO 18 or SAPO 34 or a mixture thereof.

**With regards to Me,** it is advantageously a metal selected from the group consisting of silicon, germanium, magnesium, zinc, iron, cobalt, nickel, manganese, chromium and mixtures thereof. Preferred metals are silicon, magnesium and cobalt with silicon or germanium being especially preferred.

**With regards to the TIA,** mention may be made, by way of example, of 1,2-propanediol, 1,3-propanediol, methanol, ethanol, propanol, isopropanol, butanol, glycerol or ethylene glycol.

**With regards to the organic templating agent,** it can be any of those heretofore proposed for use in the synthesis of conventional zeolitic aluminosilicates and microporous aluminophosphates. In general these compounds contain elements of Group VA of the Periodic Table of Elements, particularly nitrogen, phosphorus, arsenic and antimony, preferably N or P and most preferably N, which compounds also contain at least one alkyl or aryl group having from 1 to 8 carbon atoms. Particularly preferred nitrogen-containing compounds for use as templating agents are the amines and quaternary ammonium compounds, the latter being represented generally by the formula $R_4N^+$ wherein each R is an alkyl or aryl group containing from 1 to 8 carbon atoms. Polymeric quaternary ammonium salts such as $[(C_{14}H_{32}N_2)(OH)_2]_x$ wherein "x" has a value of at least 2 are also suitably employed. Both mono-, di and tri-amines are advantageously utilized, either alone or in combination with a quaternary ammonium compound or other templating compound. Representative templating agents include tetramethylammonium, tetraethylammonium, tetrapropylammonium or tetrabutylammonium cations; di-n-propylamine, tripropylamine, triethylamine; diethylamine, triethanolamine; piperidine; morpholine; cyclohexylamine; 2-methylpyridine; N,N-dimethylbenzylamine; N,N-diethylethanolamine; dicyclohexylamine; N,N-dimethylethanolamine; choline; N,N'-dimethylpiperazine; 1,4-diazabicyclo(2,2,2)octane; N-methyldiethanolamine, N-methylethanolamine; N-methylpiperidine; 3-methylpiperidine; N-methylcyclohexylamine; 3-methylpyridine; 4-methylpyridine; quinuclidine; N,N'-dimethyl-1,4-diazabicyclo(2,2,2)octane ion; di-n-butylamine, neopentylamine; din-pentylamine; isopropylamine; t-butylamine; ethylenediamine; pyrrolidine; and 2-imidazolidone. Advantageously organic templating agent is selected among tetraethylammonium hydroxide (TEAOH), diisopropylethylamine (DPEA), tetraethyl ammonium salts, cyclopentylamine, aminomethyl cyclohexane, piperidine, triethylamine, diethylamine, cyclohexylamine, triethyl hydroxyethylamine, morpholine, dipropylamine, pyridine, isopropylamine di-n-propylamine, tetra-n-butylammonium hydroxide, diisopropylamine, di-n-propylamine, n- butylethylamine, di-n-butylamine, and di-n-pentylamine and combinations thereof. Preferably the template, is a tetraethyl ammonium compound selected from the group of tetraethyl ammonium hydroxide (TEAOH), tetraethyl ammonium phosphate, tetraethyl ammonium fluoride, tetraethyl ammonium bromide, tetraethyl ammonium chloride, tetraethyl ammonium acetate. Most preferably, the template is tetraethyl ammonium hydroxide.

**With regards to the reactive inorganic source of $MeO_2$ essentially insoluble in the TIA and relating to silicon,** non-limiting examples of useful inorganic silicon source materials non-soluble in alcohols include, fumed silica, aerosol, pyrogenic silica, precipitated silica and silica gel.

**With regards to the reactive sources of $Al_2O_3$,** it can be any aluminum species capable of being dispersed or dissolved in an aqueous synthesis solution. Useful sources of alumina are one or more sources selected from the group consisting of the following: hydrated alumina, organo alumina, in particularly $Al(OiPr)_3$, pseudo-boehmite, aluminum hydroxide, colloidal alumina, aluminium halides, aluminium carboxylates, aluminium sulfates and mixtures thereof.

**With regards to the reactive sources of $P_2O_5$,** it can be one or more sources selected from the group consisting of phosphoric acid; organic phosphates, such as triethyl phosphate, tetraethyl-ammonium phosphate; aluminophosphates; and mixtures thereof. The phosphorous source should also be capable of being dispersed or dissolved in an alcohol synthesis solution.

**With regards to the step b)**, the reaction mixture obtained by mixing the reactive sources of alumina, $MeO_2$, phosphorus, organic templating agent and TIA is submitted to autogenous pressure and elevated temperature. The reaction mixture is heated up to the crystallization temperature that may range from about 120°C. to 250°C., preferably from 130°C. to 225°C., most preferably from 150°C. to 200°C. Heating up to the crystallization temperature is typically carried for a period of time ranging from about 0,5 to about 16 hours, preferably from about 1 to 12 hours, most preferably from about 2 to 9 hours. The temperature may be increased stepwise or continuously. However, continuous heating is preferred. The reaction mixture may be kept static or agitated by means of tumbling or stirring the reaction vessel during hydrothermal treatment. Preferably, the reaction mixture is tumbled or stirred, most preferably stirred. The temperature is then maintained at the crystallization temperature for a period of time ranging from 2 to 200 hours. Heat and agitation is applied for a period of time effective to form crystalline product. In a specific embodiment, the reaction mixture is kept at the crystallization temperature for a period of from 16 to 96 hours.

**With regards to the step c)**, the usual means can be used. Typically, the crystalline molecular sieve product is formed as a slurry and can be recovered by standard means, such as by sedimentation, centrifugation or filtration.

**With regards to the step d)**, the separated molecular sieve product is washed, recovered by sedimentation, centrifugation or filtration and dried.

**With regards to the step e)**, calcination of molecular sieves is known per se. As a result of the molecular sieve crystallization process, the recovered molecular sieve contains within its pores at least a portion of the template used. In a preferred embodiment, activation is performed in such a manner that the template is removed from the molecular sieve, leaving active catalytic sites with the microporous channels of the molecular sieve open for contact with a feedstock. The activation process is typically accomplished by calcining, or essentially heating the molecular sieve comprising the template at a temperature of from 200 to 800° C in the presence of an oxygen-containing gas. In some cases, it may be desirable to heat the molecular sieve in an environment having a low oxygen concentration. This type of process can be used for partial or complete removal of the template from the intracrystalline pore system.

Additionally, if during the synthesis alkaline or alkaline earth metals have been used, the molecular sieve might be subjected to an ion-exchange step. Conventionally, ion-exchange is done in aqueous solutions using ammonium salts or inorganic acids.

Once the molecular sieve is made, it can be used as itself as a catalyst. In another embodiment it can be formulated into a catalyst by combining the molecular sieve with other materials that provide additional hardness or catalytic activity to the finished catalyst product. The present invention also relates to catalysts consisting of the above MeAPO molecular sieves made by the method of the invention or comprising the above MeAPO molecular sieves made by the method of the invention.

Materials which can be blended with the molecular sieve can be various inert or catalytically active materials, or various binder materials. These materials include compositions such as kaolin and other clays, various forms of rare earth metals, alumina or alumina sol, titania, zirconia, quartz, silica or silica sol, and mixtures thereof. These components are effective in densifying the catalyst and increasing the strength of the formulated catalyst. When blended with non-metalloaluminophosphate molecular sieve materials, the amount of MeAPO of the present invention, which is contained in the final catalyst product ranges from 10 to 90 weight percent of the total catalyst, preferably 20 to 70 weight percent of the total catalyst.

The MeAPO molecular sieves synthesized in accordance with the present method can be used to dry gases and liquids; for selective molecular separation based on size and polar properties; as ion-exchangers; as catalysts in cracking, hydrocracking, disproportionation, alkylation, isomerization, oxidation; as chemical carriers; in gas chromatography; and in the petroleum industry to remove normal paraffins from distillates. More precisely they are useful as catalysts in a variety of processes including cracking of, for example, a naphtha feed to light olefin(s) or higher molecular weight (MW) hydrocarbons to lower MW hydrocarbons; hydrocracking of, for example, heavy petroleum and/or cyclic feedstock; isomerization of, for example, aromatics such as xylene; polymerization of, for example, one or more olefin(s) to produce a oligomer product; dewaxing of, for example, hydrocarbons to remove straight chain paraffins; absorption of, for example, alkyl aromatic compounds for separating out isomers thereof; oligomerization of, for example, straight and branched chain olefin(s); and the synthesis of monoalkylamines and dialkylamines.

The MeAPO made by the method of the present invention are particularly suited for the catalytic conversion of oxygen-containing, halogenide-containing or sulphur-containing organic compounds to hydrocarbons. Accordingly, the present invention also relates to a method for making an olefin product from an oxygen-containing, halogenide-containing or sulphur-containing organic feedstock wherein said oxygen-containing, halogenide-containing or sulphur-containing organic feedstock is contacted with the catalyst of this invention comprising the molecular sieve of this invention under conditions effective to convert the oxygen-containing, halogenide-containing or sulphur-containing organic feedstock to olefin products. In this process a feedstock containing an oxygen-containing, halogenide-containing or sulphur-containing organic compound contacts the above described catalyst in a reaction zone of a reactor at conditions effective to produce light olefins, particularly ethylene and propylene. Typically, the oxygen-containing, halogenide-containing or sulphur-containing organic feedstock is contacted with the catalyst when the oxygen-containing, halogenide-containing or sulphur-containing organic compounds is in vapour phase. Alternately, the process may be carried out in a liquid or a mixed vapour/liquid phase. In this process, converting oxygen-containing, halogenide-containing or sulphur-containing organic compounds, olefins can generally be produced at a wide range of temperatures. An effective operating temperature range can be from about 200° C. to 700° C. At the lower end of the temperature range, the formation of the desired olefin products may become markedly slow. At the upper end of the temperature range, the process may not form an optimum amount of product. An operating temperature of at least 300° C, and up to 575° C is preferred.

The pressure also may vary over a wide range. Preferred pressures are in the range of about 5 kPa to about 5 MPa, with the most preferred range being of from about 50 kPa to about 0.5 MPa. The foregoing pressures refer to the partial pressure of the oxygen-containing, halogenide-containing, sulphur-containing organic compounds and/or mixtures thereof.

The process can be carried out in any system using a variety of transport beds, although a fixed bed or moving bed system could be used. Advantageously a fluidized bed is used. It is particularly desirable to operate the reaction process at high space velocities. The process can be conducted in a single reaction zone or a number of reaction zones arranged in series or in parallel. Any standard commercial scale reactor system can be used, for example fixed bed, fluidised or moving bed systems. The commercial scale reactor systems can be operated at a weight hourly space velocity (WHSV) of from 0.1 hr$^{-1}$ to 1000 hr$^{-1}$.

One or more inert diluents may be present in the feedstock, for example, in an amount of from 1 to 95 molar percent, based on the total number of moles of all feed and diluent components fed to the reaction zone. Typical diluents include, but are not necessarily limited to helium, argon, nitrogen, carbon monoxide, carbon dioxide, hydrogen, water, paraffins, alkanes (especially methane, ethane, and propane), aromatic compounds, and mixtures thereof. The preferred diluents are water and nitrogen. Water can be injected in either liquid or vapour form.

The oxygenate feedstock is any feedstock containing a molecule or any chemical having at least an oxygen atom and capable, in the presence of the above MeAPO catalyst, to be converted to olefin products. The oxygenate feedstock comprises at least one organic compound which contains at least one oxygen atom, such as aliphatic alcohols, ethers, carbonyl compounds (aldehydes, ketones, carboxylic acids, carbonates, esters and the like). Representative oxygenates include but are not necessarily limited to lower straight and branched chain aliphatic alcohols and their unsaturated counterparts. Examples of suitable oxygenate compounds include, but are not limited to: methanol; ethanol; n-propanol; isopropanol; $C_4$-$C_{20}$ alcohols; methyl ethyl ether; dimethyl ether; diethyl ether; di-isopropyl ether; formaldehyde; dimethyl carbonate; dimethyl ketone; acetic acid; and mixtures thereof. Representative oxygenates include lower straight chain or branched aliphatic alcohols, their unsaturated counterparts.

Analogously to these oxygenates, compounds containing sulphur or halides may be used. Examples of suitable compounds include methyl mercaptan; dimethyl sulfide; ethyl mercaptan; di-ethyl sulfide; ethyl monochloride; methyl monochloride, methyl dichloride, n-alkyl halides, n-alkyl sulfides having n-alkyl groups of comprising the range of from about 1 to about 10 carbon atoms; and mixtures thereof. Preferred oxygenate compounds are methanol, dimethyl ether, or a mixture thereof.

The method of making the olefin products from an oxygenate feedstock can include the additional step of making the oxygenate feedstock from hydrocarbons such as oil, coal, tar sand, shale, biomass and natural gas. Methods for making oxygen-containing, halogenide-containing, sulphur-containing-containing organic feedstocks are known in the art. These methods include fermentation to alcohol or ether, making synthesis gas, then converting the synthesis gas to alcohol or ether. Synthesis gas can be produced by known processes such as steam reforming, autothermal reforming and partial oxidization in case of gas feedstocks or by reforming or gasification using oxygen and steam in case of solid (coal, organic waste) or liquid feedstocks. Methanol, methylsulfide and methylhalides can be produced by oxidation of methane with the help of dioxygen, sulphur or halides in the corresponding oxygen-containing, halogenide-containing or sulphur-containing organic compound.

One skilled in the art will also appreciate that the olefin products made by the oxygenate-to-olefin conversion reaction using the molecular sieve of the present invention can be polymerized to form polyolefins, particularly polyethylenes and polypropylenes.

[Examples]

In the following examples :

EG means ethylene glycol,
Eth means ethanol,
MeOH means methanol,
XRD means X ray diffraction,
SEM means scanning electron microscopy,
Aerosil 200® is a fumed silica supplied by Degussa.

Examples 1-3

A reaction mixture of TIA, phosphoric acid (85% in water) and TEAOH solution (40 % in water) was prepared in a teflon vessel. In this solution were added corresponding amount of Al source and Si-source respectively. This slurry was mixed until homogeneous for about 30 min at room temperature. Then the teflon vessel was put into a stainless autoclave. This autoclave was kept under temperature. After cooling to room temperature, a sample was taken, washed and dried. Separation of the solid from the liquid phase after synthesis was performed by centrifugation. Separated solid was dried at 110°C overnight and calcined in air flow at 600°C for 10h. Proportions and operating conditions are in the following table. This procedure was applied for all the examples.

| Example | 1 | 2 |
|---|---|---|
| molar composition | 1 TEAOH / 0.1 SiO$_2$ / 1 P$_2$O$_5$ / 1 Al$_2$O$_3$ / 12 TIA | |
| TEAOH (35% in water), g | 7.01 | 7.04 |

(continued)

| Example | 1 | 2 |
|---|---|---|
| Al isopropoxide 98%, g | 6.95 | 6.94 |
| TIA, g | 12.41 EG | 9.22 Eth |
| Aerosil 200, g | 0.10 | 0.11 |
| $H_3PO_4$ (85% in water), g | 3.84 | 3.96 |
| Conditions | 160°C, 3 days | |
| XRD | SAPO-18 | SAPO-18 |
| SEM | Lamellar Fig. 1 | Lamellar |
| Example | 3 | |
| molar composition | 1 TEAOH / 0.1 $SiO_2$ / 0.9 $P_2O_5$ / 0.9 $Al_2O_3$ / 12 TIA | |
| TEAOH (40% in water), g | 28.03 | |
| Al isopropoxide 98%, g | 27.82 | |
| TIA, g | 27.81 MeOH | |
| Aerosil 200, g | 0.50 | |
| $H_3PO_4$ (85% in water), g | 15.80 | |
| Conditions | 160°C, 3 days | |
| XRD | SAPO-18 | |
| SEM | Lamellar | |

Examples 4-6

| Example | 4 | |
|---|---|---|
| molar composition | 1 TEAOH / 0.3 $SiO_2$ / 1 $P_2O_5$ / 1 $Al_2O_3$/ 12 TIA | |
| TEAOH (35% in water), g | 28.00 | |
| Al isopropoxide 98%, g | 27.80 | |
| TIA, g | 50.15 EG | |
| Aerosil 200 g | 1.34 | |
| $H_3PO_4$ (85% in water), g | 15.30 | |
| Conditions | 3 days, 160°C | |
| XRD | SAPO-18 | |
| SEM | Lamellar | |
| Example | 5 | 6 |
| molar composition | 1 TEAOH / 0.3 $SiO_2$ / 0.9 $P_2O_5$ / 0.9 $Al_2O_3$/ 12 TIA | |
| TEAOH (40% in water), g | 28.03 | 28.03 |
| Al isopropoxide 98%, g | 27.82 | 27.82 |
| TIA, g | 39.99 Eth | 27.81 MeOH |
| Aerosil 200 g | 1.50 | 1.50 |

(continued)

| Example | 5 | 6 |
|---|---|---|
| $H_3PO_4$ (85% in water), g | 15.80 | 15.80 |
| Conditions | 3 days, 160°C | |
| XRD | SAPO-18 | SAPO-34 |
| SEM | Lamellar | Lamellar |

Examples 7-8

| Example | 7 |
|---|---|
| molar composition | 1 TEAOH / 0.6 $SiO_2$ / 1 $P_2O_5$ / 1 $Al_2O_3$/ 12 TIA |
| TEAOH (35 % in water), g | 28.10 |
| Al isopropoxide 98%, g | 27.80 |
| TIA g | 50.08 EG |
| Aerosil 200, g | 2.50 |
| $H_3PO_4$ (85% in water), g | 15.30 |
| Conditions | 3 days, 160°C |
| XRD | SAPO-18 |
| SEM | Lamellar |

| Example | 8 |
|---|---|
| molar composition | 1 TEAOH / 0.6 $SiO_2$ / 0.9 $P_2O_5$ / 0.9 $Al_2O_3$/ 12 TIA |
| TEAOH (40% in water), g | 28.03 |
| Al isopropoxide 98%, g | 27.82 |
| TIA g | 39.99 Eth |
| Aerosil 200, g | 3.06 |
| $H_3PO_4$ (85% in water), g | 15.80 |
| Conditions | 3 days, 160°C |
| XRD | SAPO-34 |
| SEM | Lamellar Fig. 2 |

Example 9

Synthesis at higher temperature

| Example | 9 |
|---|---|
| molar composition | 1 TEAOH / 0.3 $SiO_2$ / 1 $P_2O_5$ / 1 $Al_2O_3$/ 12 TIA |
| TEAOH (35% in water), g | 28.10 |
| Al isopropoxide 98%, g | 27.80 |

(continued)

| Example | 9 |
|---|---|
| TIA g | 50.08 EG |
| Aerosil 200 g | 1.34 |
| $H_3PO_4$ (85% in water), g | 15.50 |
| Conditions | 3 days, 190°C |
| XRD | SAPO-18 |
| SEM | Lamellar |

Examples 10-11

Reduced amount of TIA

| Example | 10 | 11 |
|---|---|---|
| molar composition | 1 TEAOH / 0.1 $SiO_2$ / 1 $P_2O_5$ / $Al_2O_3$ / 6 TIA | |
| TEAOH (35% in water), g | 14.02 | 11.06 |
| Al isopropoxide 98%, g | 13.89 | 10.91 |
| TIA g | 12.54 EG | 7.25 Eth |
| Aerosil 200, g | 0.20 | 0.16 |
| $H_3PO_4$ (85% in water), g | 7.69 | 6.12 |
| XRD | SAPO-18 | SAPO-18 |
| SEM | Lamellar | Lamellar |

Example 12

Synthesis with reduced amount of template

| Example | 12 |
|---|---|
| molar composition of gel | 0.7 / TEAOH / 0.1$SiO_2$ / 1$Al_2O_3$ / 15EG / 1$P_2O_5$ |
| TEAOH (35% in water), g | 9.81 |
| Al isopropoxide 98%, g | 13.89 |
| TIA, g | 31.35 EG |
| Aerosil 200, g | 0.20 |
| $H_3PO_4$ (85% in water), g | 7.69 |
| Conditions | 160°C, 4 days |
| XRD | SAPO-18 |
| SEM | Lamellar |

Example 13

Synthesis with increased amount of template in presence of EG

| Example | 13 |
|---|---|
| molar composition | $2\,TEAOH\,/\,0.1\,SiO_2\,/\,1Al_2O_3\,/\,1\,P_2O_5\,/\,6EG$ |
| TEAOH (35% in water), g | 28.00 |
| Al isopropoxide 98%, g | 13.90 |
| TIA, g | 12.54 EG |
| Aerosil 200, g | 0.20 |
| $H_3PO_4$ (85% in water), g | 7.69 |
| Conditions | 160°C, 4 days |
| XRD | SAPO-18 |
| SEM | Lamellar Fig. 3 |

Example 14

Synthesis at lower Si-content

| Example | 14 |
|---|---|
| molar composition | $1TEAOH\,/\,0.05\,SiO_2\,/\,1Al_2O_3\,/\,1\,P_2O_5\,/\,12EG$ |
| TEAOH (35 % in water), g | 14.00 |
| Al isopropoxide 98%, g | 13.90 |
| TIA, g | 25.08 EG |
| Aerosil 200, g | 0.10 |
| $H_3PO_4$ (85% in water), g | 7.7 |
| Conditions | 160°C, 4 days |
| XRD | SAPO-18 |
| SEM | Lamellar |

Comparative example I

The essential of this recipe: the source of Si must be soluble in alcohol. In the present invention all Si sources are not soluble in TIA.

Synthesis of SAPOs in presence of alcohol with organic source of Si according to US 6 540 970 protocol :

| Example | comparative example I |
|---|---|
| Recipe | US 6 540 970 B1 |
| molar composition | $2\,TEAOH\,/\,0.1\,SiO_2\,/\,1Al_2O_3\,/\,1\,P_2O_5\,/\,50\,H_2O\,/\,8\,Eth$ |
| $H_2O$, g | 19.90 |
| TEAOH (35% in water), g | 60.00 |

(continued)

| Example | comparative example I |
|---|---|
| Al source (catapal B), g a hydrated alumina | 10.04 |
| Ethanol, g | 26.28 |
| Si source TEOS, g | 1.52 |
| $H_3PO_4$ (85% in water), g | 16.44 |
| Conditions | 195°C, 1 day |
| XRD | SAPO-34/18 |
| SEM | Cubic crystal Fig. 4 |

Morphology of the samples synthesized according to this recipe is different from lamellar. Indeed, a very particular spheroidal morphology has been described in this patent for SAPO-34 sample. The crystallites have a width, at their largest dimension, of from about 0.5μm to about 30μm.
Reproduction of example for SAPO-18 synthesis led to materials with cubic crystals.

Comparative example II

Synthesis of SAPO-18 (Chen's recipe)

- Verified Syntheses of Zeolitic Materials, H. Robson, Elsevier, p.81,
- Catalysis Letters 28 (1994) 241-248
- J. Chem. Soc., Chem. Comm., 1994, 603-604
- J. Phys. Chem. 1994, 98, 10216-10224

| Example | Comparative ex II |
|---|---|
| Molar composition | $0.4SiO_2$: $1Al_2O_3$: $0.9P_2O_5$: $50H_2O$ : 1.9 DPEA |
| $H_2O$, g | 66.92 |
| $H_3PO_4$ (85% in water), g | 16.73 |
| Al source (catapal B), g a hydrated alumina | 11.35 |
| Aerosil 200, g | 1.96 |
| DPEA, g | 20.00 |
| Conditions: | 160°C, 7 days |
| XRD | SAPO-18 |
| SEM | cubes Fig. 5 |

Comparative example III

Synthesis of SAPOs according to recipe of US 6 334 994 at high and low Si content.

| Example | Comparative ex IIIa | Comparative ex IIIb |
|---|---|---|
| Recipe reference | US 6 334 994 | Microporous Mesoporous 1999,29,159 |
| molar composition | $0.075\,SiO_2\,/\,Al_2O_3\,/\,0.98\,P_2O_5\,/\,2\,TEAOH$ | $0.3\,SiO_2\,/\,Al_2O_3\,/\,0.98\,P_2O_5\,/\,2\,TEAOH$ |

(continued)

| Example | Comparative ex IIIa | Comparative ex IIIb |
|---|---|---|
| $H_2O$, g | 18.06 | 36.08 |
| Al isopropoxide 98%, g | 13.80 | 27.23 |
| $H_3PO_4$ (85% in water), g | 7.52 | 15.17 |
| HCl, g | 0.12 | 0.20 |
| Ludox AS 40 (40 % silica), g | 0.54 | 4.00 |
| TEAOH (35% in water), g | 28.20 | 56.08 |
| XRD | SAPO-18 | SAPO-34 |
| SEM | laminated cubes Fig. 6 | cubes Fig. 7 |

Comparative example IV (US 6 953 767 B2)

Inventors in the US 6953767B2 described a synthesis of SAPOs phase mixed structure. 18/34 phase ratio was tuned by changing the turning rate of autoclave during the synthesis.

The results showed, that phase composition is reproducible but the morphology was not lamellar.

| Example | Comparative ex IV same as ex 1 of US 6 953 767 B2 |
|---|---|
| molar composition | 0.15 $SiO_2$ / 1$Al_2O_3$ / 1 $P_2O_5$ / 1TEAOH / 35 $H_2O$ |
| Conditions | 175°C, 8h |
| rotation rate, rpm | 60 |
| $H_2O$, g | 32.13 |
| Alumina (Condea Pural SB), g | 19.85 |
| $H_3PO_4$, (85% in water), g | 33.55 |
| Ludox AS 40 (40 % silica), g | 3.32 |
| TEAOH (35% in water), g | 61.40 |
| TOTAL weight, g | 150.25 |
| XRD | AEI/CHA~0.2 |
| SEM | laminated cubes Fig. 8 |

Example 15 (MTO)

Catalyst tests were performed on 2g catalyst samples with a essentially pure methanol feed at 450°C, at a pressure of 0,5 barg and WHSV=1.6h$^{-1}$, in a fixed-bed, down flow stainless-steel reactor. Catalyst powders was pressed into wafers and crushed to 35-45 mesh particles. Prior to catalytic run all catalysts were heated in flowing $N_2$ (5 Nl/h) up to the reaction temperature. Analysis of the products has been performed on-line by a gas chromatograph equipped with a capillary column. Catalytic performances of MeAPOs molecular sieves were compared at 100% of methanol conversion and maximum of catalyst activity just before appearance of DME in the effluent. The results are in table 1 hereunder. The values in table 1 are the effluent of the MTO reactor and are the weight percent on carbon basis.

Table 1

| | SAPO-18 | SAPO-18 | SAPO-18 | SAPO-18 | SAPO-34/18 | SAPO-18 |
|---|---|---|---|---|---|---|
| Morphology | lamellar | lamellar | lamellar | lamellar | cubic | cubic |
| catalyst of ex No | Ex 1 | Ex 5 | Ex 10 | Ex 14 | comp ex I | comp ex II |
| methane in the effluent | 2.8 | 1.7 | 2.9 | 2.8 | 4.7 | 4.9 |

(continued)

|  | SAPO-18 | SAPO-18 | SAPO-18 | SAPO-18 | SAPO-34/18 | SAPO-18 |
|---|---|---|---|---|---|---|
|  |  |  |  |  |  |  |
| Paraffins (comprises the C1 above) | 8.7 | 3.6 | 5.4 | 8.2 | 10.1 | 9.3 |
| Olefins | 90.7 | 95.9 | 94.2 | 91.0 | 85.4 | 85.9 |
| Dienes | 0.4 | 0.2 | 0.4 | 0.2 | 3.8 | 4.2 |
| Aromatics | 0.1 | 0.3 | 0.1 | 0.5 | 0.7 | 0.6 |
|  |  |  |  |  |  |  |
| Purity C2's | 99 | 99 | 99 | 98 | 95 | 97 |
| Purity C3's | 99 | 99 | 99 | 99 | 97 | 97 |
| C3/C2 | 1.2 | 1.2 | 1.2 | 1.3 | 1.0 | 1.0 |
| C2+C3 | 70.4 | 72.7 | 74.3 | 73.2 | 73.2 | 70.8 |
|  |  |  |  |  |  |  |
| ethylene | 32.1 | 33.5 | 34.1 | 31.7 | 36.0 | 35.1 |
| propylene | 38.3 | 39.2 | 40.2 | 41.5 | 37.3 | 35.7 |

Example 16

Catalyst tests were performed on 2g catalyst samples with a methanol/$H_2O$: 70/30 feed at 450°C, at a pressure of 0,2 barg, WHSV=2.9 $h^{-1}$, in a fixed-bed, down flow stainless-steel reactor. Catalyst powders was pressed into wafers and crushed to 35-45 mesh particles. Prior to catalytic run all catalysts were heated in flowing $N_2$ (5 Nl/h) up to the reaction temperature. Analysis of the products has been performed on-line by a gas chromatograph equipped with a capillary column. Catalytic performances of SAPOs molecular sieves were compared at 100% of methanol conversion and maximum of catalyst activity just before appearance of DME in the effluent. The results are in table 2 hereunder. The values in table 2 are the effluent of the MTO reactor and are the weight percent on carbon basis.

Table 2

|  | SAPO-18 |  | SAPO-34 |
|---|---|---|---|
| Morphology | lamellar |  | laminated cube |
|  | Ex 12 |  | comp ex IIIa |
|  | 81/742 |  | 81/752 |
| Methane in the effluent | 2.0 |  | 2.5 |
|  |  |  |  |
| Purity C2's | 100 |  | 100 |
| Purity C3's | 100 |  | 98 |
| C3/C2 | 1.2 |  | 1.0 |
| C2+C3 | 78.0 |  | 80.0 |
|  |  |  |  |
| ethylene | 36.0 |  | 41.0 |
| propylene | 42.0 |  | 39.0 |

## Claims

1.    Method for preparing metalloaluminophosphate (MeAPO) molecular sieve said method comprising :

a) forming a reaction mixture containing a texture influencing agent (TIA), an organic templating agent (TEMP), at least a reactive inorganic source of $MeO_2$ insoluble in the TIA, reactive sources of $Al_2O_3$ and $P_2O_5$,

b) crystallizing the above reaction mixture thus formed until crystals of the metalloaluminophosphate are formed,

c) recovering a solid reaction product,

d) washing it with water to remove the TIA and

e) calcinating it to remove the organic template.

2. Method according to claim 1 wherein said reaction mixture has a composition expressed in terms of molar oxide ratios of :

$TEMP/Al_2O_3$ = 0.3-5,
$MeO_2/Al_2O_3$ = 0.005-2.0,
$P_2O_5/Al_2O_3$ = 0.5-2,
$TIA/Al_2O_3$ = 3-30.

3. Method according to any one of the preceding claims wherein the ratio $TEMP/Al_2O_3$ =0.5-2.

4. Method according to any one of the preceding claims wherein the ratio $MeO_2/Al_2O_3$ =0.022-0.8.

5. Method according to any one of the preceding claims wherein the ratio $P_2O_5/Al_2O_3$ = 0.8-1.2.

6. Method according to any one of the preceding claims wherein the ratio $TIA/Al_2O_3$ = 6-20.

7. Method according to any one of the preceding claims wherein $TEMP/Al_2O_3$ = 0.5-2 ; $MeO_2/Al_2O_3$ = 0.022-0.8; $P_2O_5/Al_2O_3$ = 0.8-1.2 and $TIA/Al_2O_3$ = 6-20.

8. Method according to claim 7 wherein $TEMP/Al_2O_3$ = 0.5-2 ; $MeO_2/Al_2O_3$ = 0.022-0.7; $P_2O_5/Al_2O_3$ = 0.8-1.2 and $TIA/Al_2O_3$ = 6-20.

9. Method according to claim 8 wherein $TEMP/Al_2O_3$ = 0.7-2 ; $MeO_2/Al_2O_3$ = 0.05-0.7; $P_2O_5/Al_2O_3$ = 0.8-1.2 and $TIA/Al_2O_3$ = 6-20.

10. Method according to claim 9 wherein $TEMP/Al_2O_3$ = 0.7-2 ; $MeO_2/Al_2O_3$ = 0.05-0.6; $P_2O_5/Al_2O_3$ = 0.8-1.2 and $TIA/Al_2O_3$ = 6-20.

11. Method according to any one of the preceding claims wherein Me is silicon.

12. Method according to any one of claims 10-11 wherein the texture influencing agent (TIA) is selected among 1,2-propanediol, 1,3-propanediol, methanol, ethanol, propanol, isopropanol, butanol, glycerol or ethylene glycol.

13. MeAPO made by the method according any one of the preceding claims.

14. MeAPO according to claim 13 wherein the structure is essentially CHA or AEI or a mixture thereof.

15. MeAPO according to any one of claims 12-13 wherein the structure is essentially SAPO 18 or SAPO 34 or a mixture thereof.

16. Catalysts consisting of the MeAPO molecular sieves according to any one of claims 13 to 15 or comprising the MeAPO molecular sieves according to any one of claims 13 to 15.

17. Process for making an olefin product from an oxygenate feedstock wherein said oxygenate feedstock is contacted with the catalyst of claim 16 under conditions effective to convert the oxygenate feedstock to olefin products.

18. Process according to claim 17 wherein the oxygenate compounds are methanol, dimethyl ether, or a mixture thereof.

19. Process for making an olefin product from an organic sulphur feedstock wherein said organic sulphur feedstock is contacted with the catalyst of claim 16 under conditions effective to convert the organic sulphur feedstock to olefin products.

**20.** Process for making an olefin product from an organic halide feedstock wherein said organic halide feedstock is contacted with the catalyst of claim 16 under conditions effective to convert the organic halide feedstock to olefin products.

**21.** Process according to any one of claims 17 to 20 wherein said olefin products are fractionnated to form a stream comprising essentially ethylene and at least a part of said stream is recycled on the catalyst to increase the propylene production.

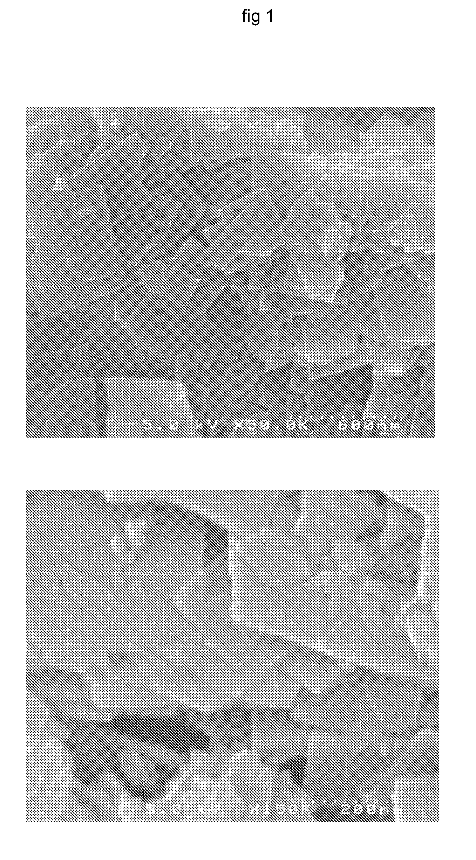

fig 1

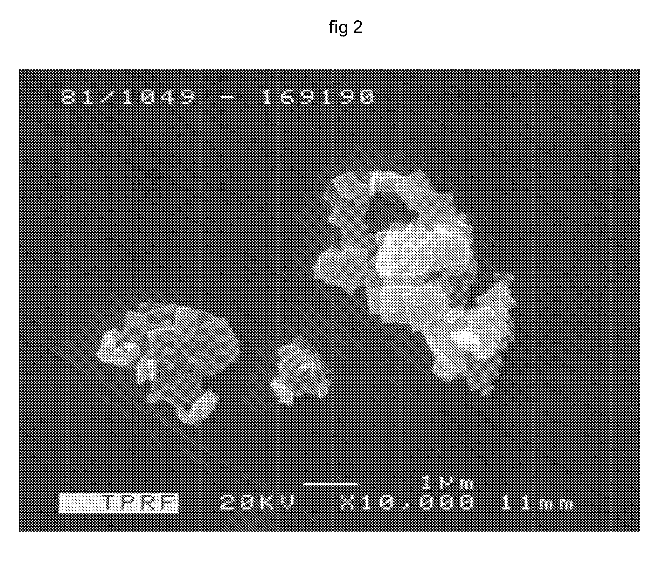

fig 2

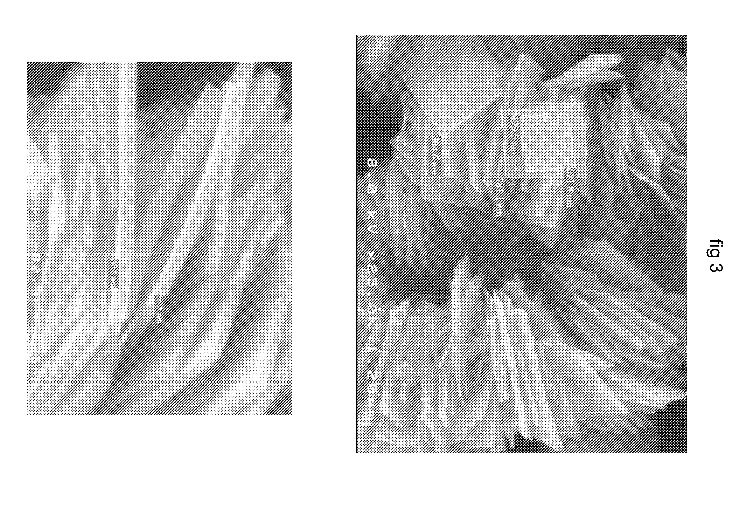

fig 3

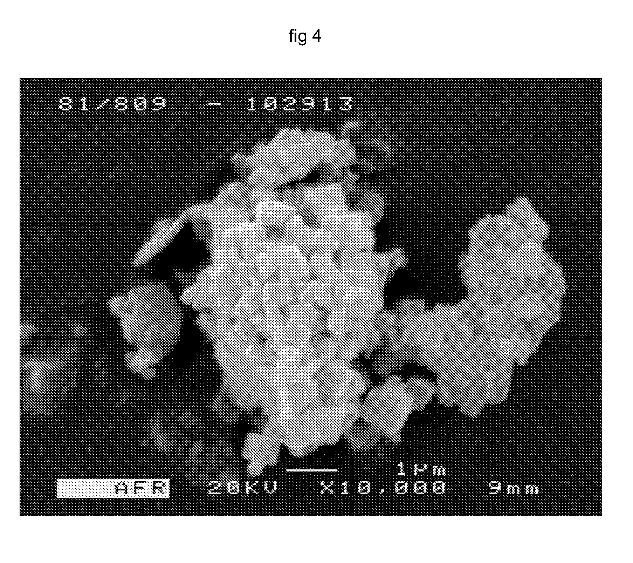

fig 4

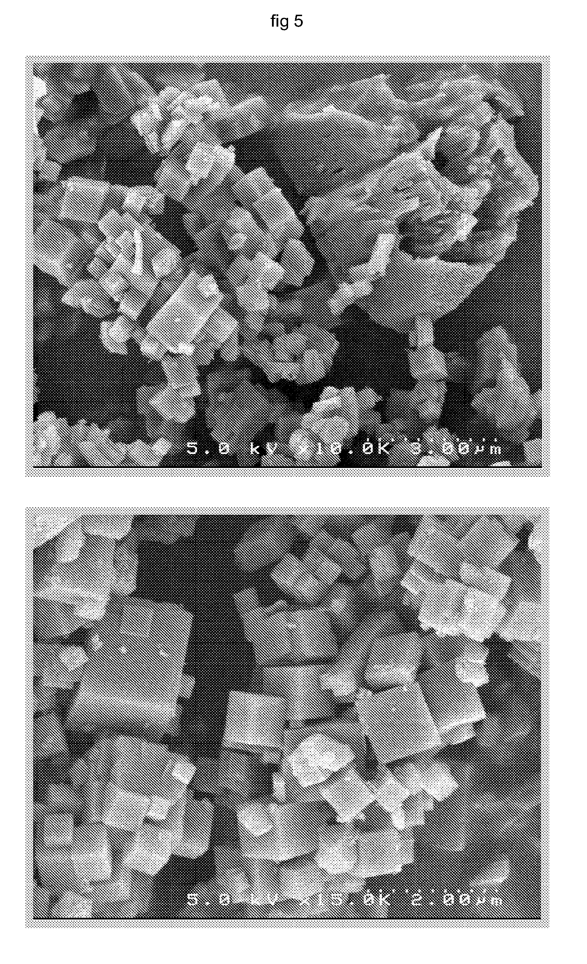

fig 5

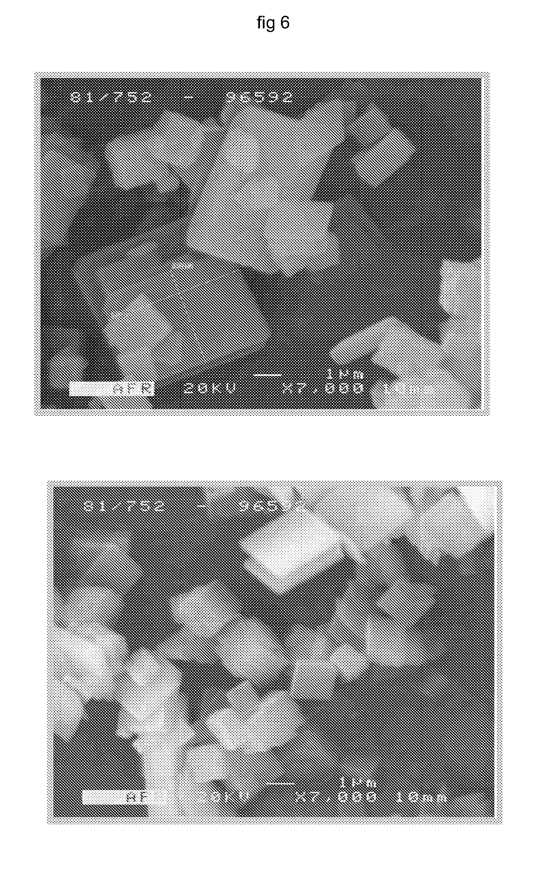

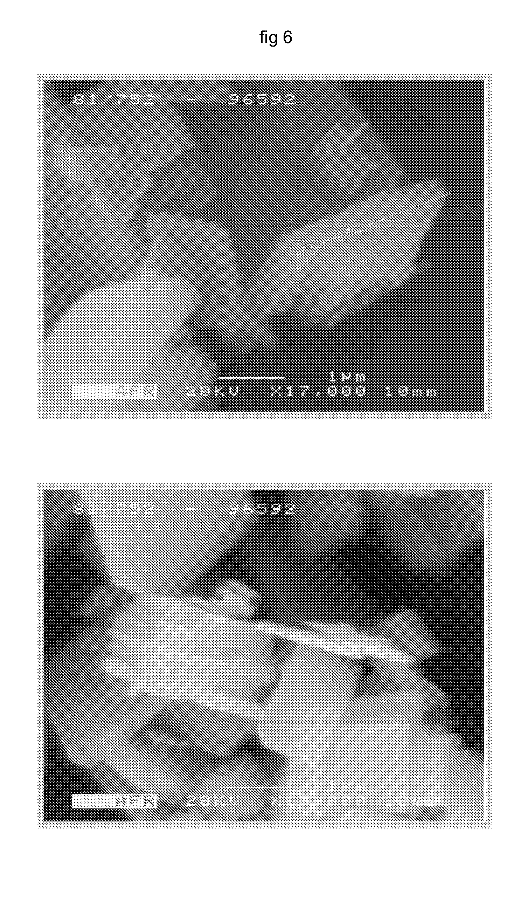

fig 6

fig 6

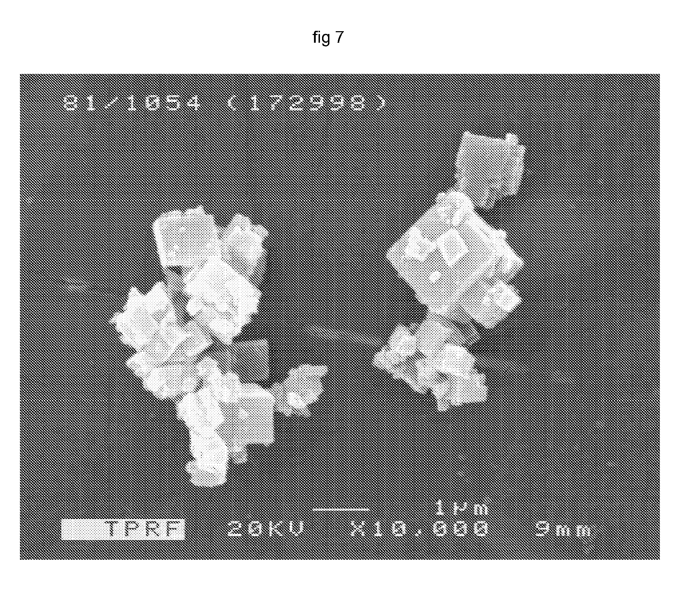

fig 7

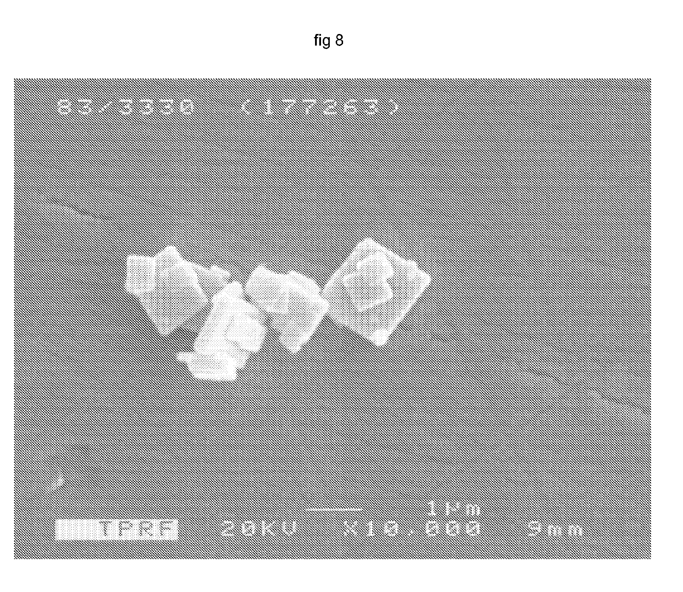

fig 8

European Patent Office

## EUROPEAN SEARCH REPORT

Application Number

EP 07 30 0861

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/147364 A1 (VENKATATHRI NARAYANAN [IN] ET AL) 6 July 2006 (2006-07-06) | 1-13,16 | INV. $C01B37/08$ |
| Y | * paragraphs [0045], [0046], [0079] *<br>----- | 14,15, 19-21 | $B01J29/85$ $C07C1/20$ $C07C1/26$ |
| X | US 2005/090390 A1 (VENKATATHRI NARAYANAN [IN] ET AL) 28 April 2005 (2005-04-28)<br><br>* paragraphs [0012] - [0014], [0030] - [0032], [0034]; claim 1 *<br>----- | 1,4, 11-13, 16-18 | $C07C1/32$ |
| X | VENKATATHRI N: "Synthesis and NMR characterization of SAPO-35 from non-aqueous systems using hexamethyleneimine template" MATERIALS RESEARCH BULLETIN, ELSEVIER, KIDLINGTON, GB, vol. 40, no. 7, 12 July 2005 (2005-07-12), pages 1157-1165, XP004914396 ISSN: 0025-5408 Experimental section: Synthesis<br>----- | 1,4 | |
| Y | US 2004/082825 A1 (BROWN STEPHEN H [BE] ET AL) 29 April 2004 (2004-04-29) * paragraphs [0029], [0035], [0039], [0042] *<br>----- | 14,15 | TECHNICAL FIELDS SEARCHED (IPC)<br>C01B<br>B01J<br>C07C |
| Y | US 4 677 243 A (KAISER STEVEN W [US]) 30 June 1987 (1987-06-30) * column 6, line 48 - line 66; example 14 *<br>----- | 19,20 | |

-/--

The present search report has been drawn up for all claims

| Place of search | Date of completion of the search | Examiner |
|---|---|---|
| Munich | 9 August 2007 | Schmitt, Ruth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................
& : member of the same patent family, corresponding document

EPO FORM 1503 03.82 (P04C01)

**European Patent Office**

**EUROPEAN SEARCH REPORT**

Application Number

EP 07 30 0861

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WU X ET AL: "Effect of feed composition on methanol conversion to light olefins over SAPO-34" APPLIED CATALYSIS A: GENERAL, ELSEVIER SCIENCE, AMSTERDAM, NL, vol. 218, no. 1-2, 25 September 2001 (2001-09-25), pages 241-250, XP004300335 ISSN: 0926-860X | 21 | |
| X | * page 244 - page 245 * ----- | 13,16-18 | |
| X | WO 02/070407 A (EXXONMOBIL CHEM PATENTS INC [US]) 12 September 2002 (2002-09-12) * page 9, line 11 - page 14, line 2 * ----- | 13-18 | |
| | | | TECHNICAL FIELDS SEARCHED (IPC) |

The present search report has been drawn up for all claims

| Place of search | Date of completion of the search | Examiner |
|---|---|---|
| Munich | 9 August 2007 | Schmitt, Ruth |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 1 970 351 A1**

## ANNEX TO THE EUROPEAN SEARCH REPORT
## ON EUROPEAN PATENT APPLICATION NO.

EP 07 30 0861

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2007

| Patent document cited in search report | | Publication date | Patent family member(s) | | Publication date |
|---|---|---|---|---|---|
| US 2006147364 | A1 | 06-07-2006 | NONE | | |
| US 2005090390 | A1 | 28-04-2005 | NONE | | |
| US 2004082825 | A1 | 29-04-2004 | NONE | | |
| US 4677243 | A | 30-06-1987 | NONE | | |
| WO 02070407 | A | 12-09-2002 | AR | 032707 A1 | 19-11-2003 |
| | | | BR | 0207788 A | 23-03-2004 |
| | | | CA | 2438146 A1 | 12-09-2002 |
| | | | CN | 1525940 A | 01-09-2004 |
| | | | EP | 1365992 A1 | 03-12-2003 |
| | | | JP | 2004524252 T | 12-08-2004 |
| | | | MX | PA03007807 A | 16-03-2004 |
| | | | NO | 20033845 A | 17-10-2003 |
| | | | TW | 583140 B | 11-04-2004 |
| | | | US | 2002165090 A1 | 07-11-2002 |
| | | | US | 2002165089 A1 | 07-11-2002 |

EPO FORM P0459

For more details about this annex : see Official Journal of the European Patent Office, No. 12/82

28

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4440871 A
- US 6207872 B
- US 6334994 B
- EP 893159 A
- US 20050096214 A

- US 6953767 B
- US 6540970 B
- US 199929159 B
- US 6953767 B2

**Non-patent literature cited in the description**

- **H. ROBSON.** Verified Syntheses of Zeolitic Materials. Elsevier, 81
- *Catalysis Letters,* 1994, vol. 28, 241-248

- *J. Chem. Soc., Chem. Comm.,* 1994, 603-604
- *J. Phys. Chem.,* 1994, vol. 98, 10216-10224